# EUROPÄISCHE PATENTANMELDUNG

(11) **EP 1 318 167 A2**
(43) Veröffentlichungstag der Anmeldung: **11.06.2003**
(21) Anmeldenummer: 02024990.0
(22) Anmeldetag: 07.11.2002
(51) Int. Cl.: C08K 3/36, C08F 2/44, C08L 23/02, C08K 9/08, A61F 2/30

(54) **Polymerbasierter Werkstoff enthaltend Silicapartikel**

(30) Priorität: 07.12.2001 DE 10160329
(71) Anmelder: MERCK PATENT GmbH, 64293 Darmstadt (DE)
(72) Erfinder: Nies, Berthold, Dr., 64407 Fränkisch-Crumbach (DE); Koch, Matthias, Dr., 65183 Wiesbaden (DE); Winkler, Holger, Dr., 64291 Darmstadt (DE)

(57) **Zusammenfassung**

Die Erfindung beschreibt ein Verfahren zur Herstellung eines polymerbasierten Werkstoffes bestehend aus einer Polyolefin-Matrix und Silicapartikel als Füllmaterial, wobei die Silicapartikel während des Polymerisationsprozesses eingebaut werden. Zusätzlich werden die Verwendungen eines polymerbasierten Werkstoffes zur Herstellung von Gelenkimplantaten im medizinisch-technischen Bereich sowie auch die Verwendung zur Herstellung von Gleitlagern, Zahnrädern und sonstigen Anwendungen im technischen Bereich beschrieben. Ferner wird ein künstliches Gelenk einer Prothese, bei dem die Gelenkpartner aus metallischen, polymeren und/oder keramischen Werkstoffen bestehen, wobei mindestens einer der Gelenkpartner aus einem polymeren Werkstoff aus Polyolefinen mit einem Zusatz von 1 bis 50Gew.% SiO₂ besteht, beschrieben.

## Beschreibung

Für künstliche Gelenke bzw. Gelenkimplantate kommen unterschiedliche Materialien und Materialkombinationen zum Einsatz, die sich vor allem in Preis und Haltbarkeit unterscheiden. Kombinationen einer geeigneten Metalllegierung mit ultrahochmolekulargewichtigem Polyethen (UHMWPE) gehören hierbei zu den preiswerten Ansätzen, die die Masse der Implantate ausmachen. Diese "metal-on-polymer"-Gelenkimplantate werden z.B. in WO 01/17464 (Depuy Int. Ltd.) beschrieben.
Ein Nachteil der Metall-UHMWPE-Kombination ist die zwischen dem Metall und dem Kunststoff bestehende Haftreibung, wodurch das Kunststoffteil verschleissanfällig ist und die Empfindlichkeit des UHMWPE gegen Einbaufehler und Beschädigung beim Einbau, z. B. durch Kontaminationen mit Knochenpartikeln oder Knochenzement.
Die Aufgabe der vorliegenden Erfindung ist daher die Bereitstellung eines polymerbasierten Werkstoffes sowie eines Verfahrens zu dessen Herstellung, welches die Nachteile des Standes der Technik zumindest teilweise dahingehend beseitigt, dass der Werkstoff in Gelenkimplantaten oder künstlichen Gelenken eine verminderte Reibung zwischen zwei Gelenkteilen ermöglicht.

Diese Aufgabe wird durch ein Verfahren zur Herstellung eines polymerbasierten Werkstoffes gemäß Anspruch 1 gelöst. Zusätzlich wird ein polymerbasierter Werkstoff gemäss den Ansprüchen 6 bis 9 beansprucht.
Ferner werden die Verwendungen eines polymerbasierten Werkstoffes zur Herstellung von Gelenkimplantaten (z.B. Hüft- und Kniegelenke) im medizinisch-technischen Bereich sowie auch die Verwendung zur Herstellung von Gleitlagern, Zahnrädern und sonstigen Anwendungen im technischen Bereich beansprucht.

Ein weiterer Gegenstand der vorliegenden Erfindung ist ein künstliches Gelenk einer Prothese, bei dem die Gelenkpartner aus metallischen, polymeren und/oder keramischen Werkstoffen bestehen, wobei mindestens einer der Gelenkpartner aus einem polymeren Werkstoff aus Polyolefinen mit einem Zusatz von 1 bis 50Gew.% SiO₂ besteht. Bevorzugt ist ein Bereich von 5 bis 30Gew. % SiO₂.

Als Polyolefin-Matrix kommen alle Arten von Polyolefinen in Frage. Bevorzugt sind jedoch Polyethylene (PE).

Als PE-Formteile werden bevorzugt UHMWPE-Formteile verwendet, welche generell ein extrem hohes Molekulargewicht zwischen 3.9 bis 10.5 Mio g/mol besitzen. Mit steigender molarer Masse verbessern sich einige technisch wichtige Eigenschaften der Polyethylene wie z.B. Verschleißfestigkeit, Kerbschlagzähigkeit, Formbeständigkeit unter Wärmeeinfluss und Gleitfähigkeit. Gleichzeitig nimmt die Verarbeitbarkeit der hochmolekularen PEs ab. Es ist daher auch ein Ziel der Erfindung, ein gut verarbeitbares PE in den Kompositen verwenden zu können, mit dem ähnlich gute oder bessere Eigenschaften zu erzielen sind, als mit den bisher gebräuchlichen PEs.

Bei Prothesen mit künstlichen Gelenken können aufgrund der erforderlichen Verträglichkeit mit dem menschlichen oder auch tierischen Gewebe, der sogenannten Biokompatibilität, und der hohen Reibbelastung der Gelenkpartner nur wenige Werkstoffe eingesetzt werden. Von den metallischen Werkstoffen hat sich vor allem Cobalt-Chrom (CoCrMo) bewährt. Anwendungen von Titan-basierten Legierungen oder Oberflächen-modifizierten Titanlegierungen (z. B. Titan-Nitrid) sind noch im Versuchsstadium. Von den Kunststoffen kommt insbesondere Polyethylen (UHMWPE) bei den Pfannen der Hüftgelenk-Endoprothesen und als Gleitpartner der Schienbeinkomponente zum Einsatz und Aluminiumoxid und Zirkonoxid sind die brauchbaren Keramikwerkstoffe.

Der Verschleiß innerhalb des künstlichen Gelenkes wird durch den Einbau von harten, unporösen SiO₂-Kugeln bzw. Silicapartikel in das PE-Formteil minimiert.

Um eine nanodisperse und möglichst homogene Verteilung der Silicapartikel im PE zu erreichen, ist es vorteilhaft, sie bereits während des Polymerisationsprozesses einzubauen.
UHMWPE wird gewöhnlich nicht extrudiert, sondern durch Heißpressen verarbeitet. Ein nachträgliches Beimengen von Füllstoffen ist dabei nicht ohne weiteres möglich.

Für den Einbau der Silicapartikel in das PE während des Polymerisationsprozesses können hierzu mit geeigneten Funktionalisierungen unterschiedliche Wege (oder Kombinationen daraus) beschritten werden. Die Silicapartikel können z.B. mit Metallocenen beladen werden und dann zur Polymerisation eingesetzt werden. Ebenso ist anstelle des Metallocenes die Beladung mit einer polymerisationsaktiven Spezies nach Ziegler-Natta (z. B. TiCl₄) oder Phillips möglich. Das PE entsteht dabei um die Teilchen herum, was eine bessere Verteilung bewirkt als nachträgliches Vermischen beim Extrusionsprozess. Dabei ist es wichtig, die Beladung so zu wählen, dass der Silicaanteil in Polyolefin zwischen 1 und 50Gew.% SiO₂ bezogen auf die Gesamtmasse des Gemisches liegen. Vorzugsweise sollte er zwischen 5 und 30Gew. % liegt. Ein zu hoher SiO₂-Gehalt (>55Gew.%) hat jedoch den Nachteil, dass das Material spröde wird und brechen kann.

Eine Funktionalisierung der Silicapartikel mit nucleophilen, nicht-aciden Verbindungen, zum Beispiel Polyethern, ermöglicht die direkte Trägerung Methylaluminoxan (MAO)-aktivierbarer Verbindungen; vornehmlich die von Metallocenen (wie in der parallelen Anmeldung der Anmelderin mit gleichem Anmeldetag beschrieben). Die funktionalisierten Partikel bilden zusammen mit MAO mittels koordinativer Wechselwirkungen ein Gel. Das Metallocen wird somit über das MAO an den Träger gebunden. Dabei wird eine homogene Verteilung von Träger und polymerisationsaktiver Spezies erreicht. Nach dem Trocknen ist der Katalysator direkt zur Polymerisation einsetzbar.
Bei Katalysatoren nach Ziegler-Natta wird die polymerisationaktive Verbindung nach allgemein bekannten Verfahren direkt (z.B. als TiCl₄-Dampf) auf die Silicateilchen aufgebracht.

Die Polymerisation wird in bekannter Weise in Lösungs-, Suspensionsoder Gasphasenpolymerisation kontinuierlich oder diskontinuierlich durchgeführt, wobei die Gasphasen- und die Suspensionspolymerisation ausdrücklich bevorzugt sind. Typische Temperaturen bei der Polymerisation liegen im Bereich von 0°C bis 200°C, vorzugsweise im Bereich von 20°C bis 140°C. Weitere Einzelheiten zum Herstellungsverfahren sind in der parallelen Anmeldung ("Mikropartikuläres Material") der Anmelderin zu finden, die den gleichen Anmeldetag trägt.

Metallocenbeladung und Katalysatoraktivierung werden so gewählt, dass eine Katalysatorproduktivität zwischen 2 und 10 (g PE)/(g Kat) erreicht wird. Dies entspricht einem Silicaanteil von 10 bis 50 Gew.%.
Üblicherweise für die Polyolefinproduktion eingesetzte Katalysatoren, die für hohe Produktivitäten ausgelegt sind, können durch "verdünnen" mit entsprechend behandelten Silicateilchen ebenfalls verwendet werden. Hierbei kommen bevorzugt olefinfunktionalisierte Partikel zum Einsatz, auf deren Oberfläche sich keine reaktiven Gruppen mehr befinden. Die Desaktivierung erfolgt zum einen durch die Olefinfunktionalisierung und darüber hinaus durch Behandeln mit Aluminiumalkylen, z.B. Diisobutylaluminiumhydrid.

Es kann auch alternativ eine Verankerung der Silicapartikel im PE durch alkylische oder olefinische Funktionen am SiO₂ erfolgen, die als Copolymere in das PE eingebaut werden um eine besonders gute Verankerung zu erreichen.
Als olefinische Funktionen werden Moleküle wie 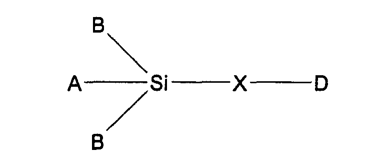 eingesetzt,
wobei **A** eine Alkoxygruppe, bevorzugt Ethoxy oder Methoxy, **B** eine ebensolche Alkoxy- oder Alkylgruppe, bevorzugt Methyl und
**D** einen Kohlenwasserstoff mit mindestens einer endständigen Alkylfunktion, bevorzugt eine lineare Alkylkette mit endständiger Doppelbindung repräsentiert.
**X** ist entweder eine direkte oder eine Heteroatom-enthaltende Verbindung wie (CH₂)ₙ-O-, wobei n zwischen 1 und 5 liegt.

Für die Alkyl-Funktionen werden Moleküle wie 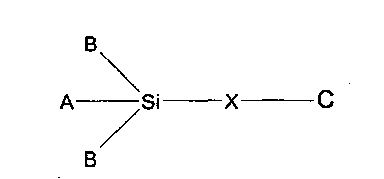 eingesetzt,
wobei **A** eine Alkoxygruppe, bevorzugt Ethoxy oder Methoxy,
**B** eine ebensolche Alkoxy- oder Alkylgruppe, bevorzugt Methyl und **C** eine Alkylkette der Länge 1-25, bevorzugt 10-20 repräsentiert.
**X** ist entweder eine direkte oder eine Heteroatom-enthaltende Verbindung wie (CH₂)ₙ-O-, wobei n zwischen 1 und 5 liegt.

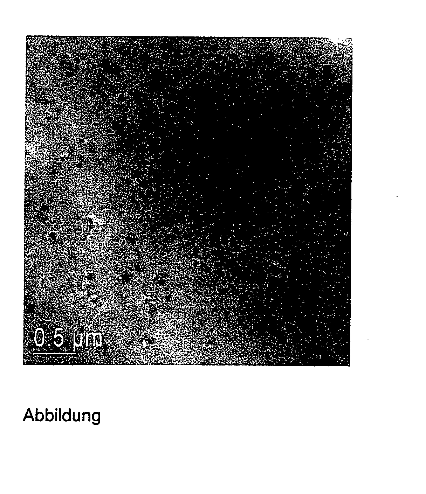

Die folgenden Beispiele und die Abbildung sollen die Erfindung näher erläutern.
Die Abbildung (siehe Anhang) zeigt eine TEM-Aufnahme (aufgenommen mit einem Transmissionselektronenmikroskop Philips CM20, Beschleunigungsspannung 200 kV) eines 8 Gew.% Silica enthaltenden PE, wobei die Probe mittels Ultramikrotomtechnologie präpariert wurde. Das Material wurde mit Hilfe von metallocenbeladenen Silicateilchen hergestellt. Katalysatorproduktivität lag bei 11g Polyethylen / g Katalysator. Deutlich ist die Verteilung der Silicateilchen zu erkennen.
Die mit nicht-aciden Nucleophilen wie Polyether, vorzugsweise PEOfunktionalisierten Monospher (=monodisperse SiO₂-Partikel) haben einen Durchmesser von 10 bis 500nm, vorzugsweise etwa 100nm. Die auf den Monospher immobilisierte Metallocenmenge wird so gewählt, dass während der Polymerisation ausreichend Polyolefin, vorzugsweise UHMWPE gebildet wird um den Silicagehalt des Produktes zwischen 1 und 50 Gew.%, vorzugsweise 5 bis 30Gew.% zu halten.

### Beispiele

### 1. Verwendung als Mikropartikuläres Material für Metallocene

2g Polyethylenoxid-funktionalisierte Monospher *(Herstellung siehe parallele Anmeldung des Anmelders "Mikropartikuläres Material" mit gleichem Anmeldetag*) mit einem Durchmesser von 100 nm wurden mit 4 ml einer Lösung von Methylaluminoxan in Toluol (w(MAO)=5%) für zwei Stunden gerührt. und mit 0,2 ml einer Lösung von Dicyclopentadienylzirconiumdichlorid in obiger MAO-Lösung (c(Zr)= 0,05 mol/l) versetzt. Nach 30 Min. weiteren Rührens wurde das Lösungsmittel im Vakuum entfernt. Zurück blieb ein gelb gefärbtes, fließendes Pulver, welches direkt zur Polymerisation eingesetzt wurde. (Beladung: 2,3 µmol Zr / g Kat, Al/Zr = 630.)

Zur Polymerisation wurde 960 mg des obigen Katalysators bei 70 °C in einen mit 400ml Isobutan gefüllten und mit Ethen auf 36 bar gebrachten 1l Stahlreaktor durch eine Schleuse mittels Argonüberdruck eingeschossen. Nach 10 Min. wurde die Polymerisation durch Ablassen des Druckes beendet. Ausbeute: 8,6 g farbloses, rieselfähiges Pulver. (Produktivität: 7,6 g PE / g Kat, Silicaanteil: 11 %.)

Das Produkt zeigt in Abriebtests nach ASTM G99 eine erhöhte Abriebfestigkeit gegenüber gewöhnlichen PE.

### 2. Verwendung als Reaktorbeimischung

■ Herstellung von C₁₈-silanisierten Monospheres
   In den 500 ml Dreihalskolben werden 132g Monospheres-Dispersion und 150g Ethanol vorgelegt. Die Mischung wird auf 70°C temperiert und unter starkem Rühren werden mittels des Tropftrichters die 12.7g ethanolischen Silanlösung über einen Zeitraum von 20 min zugetropft. Dabei ist darauf zu achten, dass die Lösung am Rand des Gefäßes eingeleitet wird, aber nicht mit dessen Wandung in Kontakt kommt.
   Danach wird die Mischung 20 h unter Rückfluss gekocht. Die Suspension, in welcher der Feststoff in zusammengeballter Form vorliegt, wird in ein Becherglas überführt. Der Feststoff wird fünfmal mit VE-Wasser dekantierend gewaschen. Nach Zugabe von Toluol wird mit einem Wasserabscheider das Wasser azeotrop aus dem Gemisch entfernt.
   Die gravimetrische Massenbestimmung ergibt eine SiO₂-Konzentration der Dispersion von 19.4 Gew.%.
■ Verwendung als Reaktorbeimischung
   In einem 11 Stahlreaktor werden 10g der oben hergestellten Octadodecylfunktionalisierte Monospher in 400ml Isobutan vorgelegt. Der Reaktor wird auf 70°C aufgeheizt und mit Ethen auf 36bar gebracht. Durch eine Schleuse werden 80mg Silicageträgertes Dicyclopentadienylzirconiumdichlorid mittels Argonüberdruck eingeschossen. Nach 60min wird die Reaktion durch Ablassen des Druckes beendet.
   Ausbeute: 78g farbloses Granulat (0.5-1 mm Teilchendurchmesser). Katalysatorproduktivität: 850g PE/ g Katalysator, Silicagehalt: 12.8 Gew.% Das Produkt zeigt in Abriebtests nach ASTM G99 eine erhöhte Abriebfestigkeit gegenüber auf identischem Wege ohne die Beimischung hergestelltem PE.

## Patentansprüche

1. Verfahren zur Herstellung eines polymerbasierten Werkstoffes bestehend aus einer Polyolefin-Matix und Silicapartikel als Füllmaterial, **dadurch gekennzeichnet, dass** die Silicapartikel bereits während des Polymerisationsprozesses eingebaut werden.

2. Verfahren nach Anspruch 1, **dadurch gekennzeichnet, dass** Polyethylenoxid-funktionalisierte Monospher mit einem Durchmesser von etwa 100 nm als Metallocenträger verwendet werden und die auf den Monospher immobilisierte Metallocenmenge so gewählt wird, dass während der Polymerisation ausreichend Polyolefin gebildet wird um den Silicagehalt des Produktes zwischen 1 und 50Gew.% zu halten.

3. Verfahren nach Anspruch 1 und/oder 2, **dadurch gekennzeichnet, dass** eine polymerisationsaktive Species vom Ziegler-Natta oder Phillips-Typ auf die Silicateilchen aufgebracht wird.

4. Verfahren nach einem der Ansprüche 1 bis 3, **dadurch gekennzeichnet, dass** einem Katalysator alkylfunktionalisierte Monospher in ausreichender Menge beigemengt werden und diese Mischung zur Polymerisation eingesetzt wird.

5. Verfahren nach einem der Ansprüche 1 bis 3, **dadurch gekennzeichnet, dass** vor der Polymerisation olefinfunktionalisierte Monospher beigemischt werden.

6. Polymerbasierter Werkstoff bestehend aus einer Polyethylen-Matrix, in der SiO₂-Partikel nanodispers und homogen verteilt sind, **dadurch gekennzeichnet, dass** die SiO₂-Partikel mit nicht-aciden Nucleophilen wie Polyether-Ketten funktionalisiert sind.

7. Polymerbasierter Werkstoff nach Anspruch 6, **dadurch gekennzeichnet, dass** die SiO₂-Partikel mit Polyethylenoxid-Ketten funktionalisiert sind.

8. Polymerbasierter Werkstoff nach Anspruch 6, **dadurch gekennzeichnet, dass** alkyl- oder olefin-funktionaliserte Silicateilchen eingesetzt werden.

9. Polymerbasierter Werkstoff erhältlich durch ein Verfahren gemäß einen der Ansprüche 1 bis 5.

10. Verwendung eines polymerbasierten Werkstoffes nach einen der Ansprüche 6 bis 9 zur Herstellung von Gelenkimplantaten im medizinisch-technischen Bereich.

11. Verwendung eines polymerbasierten Werkstoffes nach einen der Ansprüche 6 bis 9 zur Herstellung von Gleitlagern, Zahnrädern oder sonstigen Anwendungen im technischen Bereich.

12. Künstliches Gelenk einer Prothese, bei dem die Gelenkpartner aus metallischen, polymeren und/oder keramischen Werkstoffen bestehen, **dadurch gekennzeichnet, dass** mindestens einer der Gelenkpartner aus einem polymeren Werkstoff aus Polyolefinen mit einem Zusatz von 1 bis 50Gew.% SiO₂ besteht.

13. Künstliches Gelenk nach Anspruch 12, **dadurch gekennzeichnet, dass** der polymere Werkstoff aus Polyolefinen mit einem Zusatz von vorzugsweise 5 bis 30Gew.% SiO₂ besteht.

14. Künstliches Gelenk nach einem der Ansprüche 12 oder 13, **dadurch gekennzeichnet, dass** der polymere Werkstoff UHMW-Polyethylen ist.

15. Künstliches Gelenk basierend auf einem polymerbasierten Werkstoff, der erhältlich durch ein Verfahren gemäß einem der Ansprüche 1 bis 5 ist.

16. Künstliches Gelenk nach einem der Ansprüche 12 bis 15, **dadurch gekennzeichnet, dass** das Gelenk ein künstliches Hüft- oder Kniegelenk ist.
